Europäisches Patentamt

European Patent Office

Office européen des brevets

(19)

(11) Numéro de publication: **0 006 062**
**B1**

(12) **FASCICULE DE BREVET EUROPEEN**

(45) Date de publication du fascicule du brevet: **23.03.83**

(51) Int. Cl.³: **C 07 D 239/30**

(21) Numéro de dépôt: **79400342.6**

(22) Date de dépôt: **31.05.79**

(54) **Nouveau procédé de préparation de la chloro-2-pyrimidine.**

(30) Priorité: **31.05.78 GB 2565578**

(43) Date de publication de la demande:
**12.12.79 Bulletin 79/25**

(45) Mention de la délivrance du brevet:
**23.03.83 Bulletin 83/12**

(84) Etats contractants désignés:
**AT BE CH DE FR GB IT LU NL SE**

(56) Documents cités:
**FR - A - 985 292**
**US - A - 2 477 409**

**Chemical Abstracts, Volume 83, no. 15, 13 octobre 1975 (COLUMBUS, OHIO, USA) V. KRCHNAK et al. "Diazotization of the amino group on the pyrimidine nucleus" abrégé no. 131543r**
**HUOBEN-WEXL "Methoden der organischen Chemie" 4ème édition, Volume 5/3 1962, GEORG THIEME VERLAG Stuttgart, page 852**

(73) Titulaire: **Société d'Expansion Scientifique EXPANSIA Société Anonyme**
**264, rue du Faubourg Saint-Honoré**
**F-75008 Paris (FR)**

(72) Inventeur: **Aspisi, Christian**
**Les Grands Vallons Chemin du Breuil**
**F-13150 Boulbon (FR)**
Inventeur: **Demosthene, Claude**
**Route de Théziers**
**F-30390 Aramon (FR)**

(74) Mandataire: **Lachassagne, Jacques**
**Boite Postale 07**
**F-78430 Louveciennes (FR)**

Courier Press, Leamington Spa, England.

## Nouveau procédé de préparation de la chloro-2-pyrimidine

La présente invention concerne une amélioration du procédé de préparation de la chloro-2-pyrimidine.

Ce composé est déjà connu, mais, jusqu'ici, les procédés utilisés pour sa préparation n'étaient pas très satisfaisants, surtout en ce qui concerne le rendement.

L'une de ces synthèses est décrite dans "Organic Synthesis", Coll. Volume IV, page 182; selon cette méthode, on effectue la réaction entre —15°C et —10°C (utilisation d'un sel de diazonium en présence d'acide chlorhydrique); le rendement est toujours inférieur à 30%. Une légère amélioration a été décrite plus tard par KRCHNAK. U. et ARNOLD Z., Czechoslow. chem. Commun. *40*, 1390 (1975), en utilisant un chlorure de lithium 5M; le rendement était néanmoins inférieur à 50%. On a remarqué que, dans la méthode partant de l'amino-2-pyrimidine en passant par le sel de diazonium, s'il était possible d'augmenter la concentration en ions chlorure, le rendement pouvait être amélioré de façon significative. Des recherches approfondies ont montré que les chlorures qui convenaient étaient ceux des métaux de transition et de l'étain, de l'antimoine, du thallium et du zinc. Les conditions de réaction les plus favorables ont été rencontrées en utilisant du chlorure de zinc au lieu du chlorure de lithium employé précédemment, ce qui a rendu possible la réaction à une température plus élevée qu'auparavant, avec une augmentation corrélative du rendement. Le chlorure de zinc conduit à un complexe intermédiaire de meilleure stabilité, il se dissout bien dans le mélange réactionnel, il est facile à récupérer et comparativement moins cher.

En ce qui concerne les solvants à utiliser, tout solvant non polaire avec un point d'ébullition peu élevé peut convenir; on obtient de très bons résultats avec le chlorure de méthylène.

La présente invention concerne plus précisément une amélioration du procédé de préparation de la chloro-2-pyrimidine à partir d'amino-2-pyrimidine, d'acide chlorhydrique et d'un nitrite alcalin, les réactifs étant sensiblement dans les mêmes proportions que dans les méthodes précédemment connues, consistant à préparer un mélange d'acide chlorhydrique et d'un solvant non polaire à bas point d'ébullition, puis à introduire l'amino-2-pyrimidine et à ajouter lentement, à la température ambiante, un chlorure métallique choisi parmi ceux des métaux de transition, l'étain, l'antimoine, le thallium et le zinc, puis à introduire, sous circulation de gaz inerte, le nitrite alcalin, à une température n'excédant pas 10°C et enfin à verser le mélange réactionnel dans de l'eau glacée.

Selon une autre caractéristique de l'invention, le chlorure métallique utilisé est le chlorure de zinc.

L'invention sera mieux comprise grâce à la description des exemples suivants:

Exemple 1

Dans un réacteur de 250 litres, on prépare le mélange de 27 kg (environ 265 moles) d'acide chlorhydrique concentré (d=1,18) et de 35 kg—ou 28 litres—de chlorure de méthylène. On ajoute ensuite, en agitant, 8 kg (84,12 moles) d'amino-2-pyrimidine, ce qui conduit à une solution. Après avoir refroidi le mélange, on ajoute, entre 15 et 20°C, 33 kg (242,5 moles) de chlorure de zinc; on effectue cette addition en 30 minutes environ et ceci conduit à une suspension couleur moutarde.

Après avoir encore refroidi le mélange à 5 ou 10°C et fait passer un courant d'azote, on ajoute (en trois heures et quart) 10 kg (c'est-à-dire 145 moles) de nitrite de soude; il y a émission de vapeurs nitreuses; 30 minutes après la fin de cette addition, on verse le mélange réactionnel dans 150 litres d'eau glacée et on le laisse reposer.

La phase organique est ensuite séparée et la phase aqueuse est traitée 4 fois avec 50 kg (40 litres) de chlorure de méthylène. Toutes les phase organiques de lavage sont réunies et séchées sur 3 kg de sulfate de sodium sec, puis filtrées, concentrées à la pression ordinaire à 45—50°C; ceci permet de récupérer environ 80% du chlorure de méthylène.

La phase organique restante est ensuite concentrée dans un évaporateur rotatif, sous pression réduite (25 mm de Hg) à une température n'excédant pas 35°C, puis séchée pendant une heure et demie à 40°C.

On obtient 6,6 kg (rendement 69%) de chloro-2-pyrimidine, qui est une poudre jaunâtre cristalline fondant à 63—65°C (Koffler), et dont l'analyse montre une correspondance parfaite avec la formule: $C_4H_3N_2Cl$.

Exemple 2

Dans un réacteur de 600 litres, on prépare le le mélange de 54 kg d'acide chlorhydrique concentré (d=1,18) et de 70 kg (56) litres) de chlorure de méthylène.

On ajoute ensuite, en agitant, 16 kg (168,24 moles) d'amino - 2 - pyrimidine. Après avoir refroidi le mélange à 0°C, on ajoute, entre 10 et 20°C, 45 kg (200 moles) de chlorure d'antimoine. On effectue cette addition en 45 minutes, avec précaution. On obtient une suspension marron.

Après avoir encore refroidi le mélange à 5 ou 10°C, on fait passer un courant d'azote et on ajoute, en 1 heure, 20 kg (290 moles) de nitrite de soude; il y a émission de vapeurs nitreuses. Trente minutes après la fin de cette addition, on verse le milieu réactionnel dans 300 litres d'eau glacée et on le laisse reposer.

On sépare la phase organique. On lave la phase aqueuse par 80 litres de chlorure de méthylène. On sèche la phase organique par 6 kg de sulfate de sodium sec. On filtre et on concentre sous pression atmosphérique normale à 45—50°C. On récupère 75% du chlorure de méthylène recyclable.

La phase organique restante est ensuite concentrée sous pression atmosphérique réduite (environ 20 mm Hg), à une température n'excédant pas 35°C.

On obtient un solide qui est séché à 40°C pendant toute un nuit.

On obtient 11 kg (57,5%) de chloro - 2 - pyrimidine.

Exemple 3

On utilise la méthode décrite dans l'exemple 2 en remplaçant le chlorure d'antimoine par 9,7 kg de chlorure ferrique (60 moles).

Rendement: 9,9 kg (52%).

**Revendications**

1. Procédé de préparation de la chloro - 2 - pyrimidine à partir d'amino - 2 - pyrimidine, d'acide chlorhydrique et d'un nitrite alcalin, les réactifs étant sensiblement dans les mêmes proportions que dans les méthodes précédemment connues, consistant à préparer un mélange d'acide chlorhydrique et d'un solvant non polaire à bas point d'ébullition, puis à introduire l'amino - 2 - pyrimidine, caractérisé en ce que l'on ajoute lentement, à température ambiante, un chlorure métallique choisi parmi ceux des métaux de transition, de l'étain, de l'antimoine, du thallium et du zinc, puis que l'on introduit, sous circulation de gaz inerte, le nitrite alcalin, à une température n'excédant pas 10°C et qu'enfin on verse le mélange réactionnel dans de l'eau glacée.

2. Procédé de préparation selon la revendication 1, dans lequel le chlorure métallique est le chlorure de zinc.

3. Procédé de préparation selon la revendication 1 ou 2, dans lequel le solvant non polaire est le chlorure de méthylène.

**Patentansprüche**

1. Verfahren zur Herstellung von 2-Chlorpyrimidin ausgehend von 2-Aminopyrimidin, Chlorwasserstoff-säure und einem Alkalinitrit, wobei die Reaktionskomponenten im wesentlichen in den gleichen Anteilen vorhanden sind wie bei den bisher bekannten Methoden, das darin besteht, ein Gemisch von Chlorwasserstoffsäure und einem nicht-polaren Lösungsmittel mit niedrigem Siedepunkt herzustellen und anschliessend das 2-Aminopyrimidin einzuführen dadurch gekennzeichnet, dass man langsam bei Umgebungstemperatur ein Metallchlorid, ausgewählt unter denen der Übergangsmetalle, von Zinn, Antimon, Thallium und Zink, zufügt und anschliessend unter Zirkulieren eines Inertgases das Alkalinitrit bei einer Temperatur, die 10°C nicht überschreitet, einbringt und schliesslich das Reaktionsgemisch in Eiswasser giesst.

2. Herstellungsverfahren nach Anspruch 1, bei dem das Metallchlorid Zinkchlorid ist.

3. Herstellungsverfahren nach Anspruch 1 oder 2, bei dem das nicht-polare Lösungsmittel Methylenchlorid ist.

**Claims**

1. A process of preparation of 2 - chloro - pyrimidine starting from 2 - amino - pyrimidine, hydrochloric acid and an alkali metal nitrite, the reactants being present in substantially the same proportions as in previously known methods, consisting of preparing a mixture of hydrochloric acid and a non-polar solvent of low boiling point and then introducing 2 - amino - pyrimidine, characterised in that a metal chloride selected from those of transition metals, tin, antimony and zinc is added slowly at ambient temperature, the alkali metal nitrite is then introduced under an inert gas flow at a temperature not exceeding 10°C, and finally the reaction mixture is poured into iced water.

2. A process of preparation according to claim 1 in which the metal chloride is zinc chloride.

3. A process of preparation according to claim 1 or claim 2 in which the non-polar solvent is methylene chloride.